# EUROPEAN PATENT SPECIFICATION

(11) **EP 2 916 730 B1**
(45) Date of publication and mention of the grant of the patent: **18.10.2023**
(21) Application number: 13815541.1
(22) Date of filing: 07.11.2013
(51) Int. Cl.: A61B 5/291, A61B 5/259, A61B 5/00

(54) **ARRANGEMENT FOR CARRYING OUT ELECTRODE MEASUREMENTS**
ANORDNUNG ZUR DURCHFÜHRUNG VON ELEKTRODENMESSUNGEN
AGENCEMENT POUR RÉALISER DES MESURES AVEC DES ÉLECTRODES

(30) Priority: 12.11.2012 FI 20126186
(43) Date of publication of application: 16.09.2015
(73) Proprietor: Bittium Biosignals Oy, 70800 Kuopio (FI)
(72) Inventor: LAPPALAINEN, Reijo, 70870 Hiltulanlahti (FI); MERVAALA, Esa, 70110 Kuopio (FI); MYLLYMAA, Katja, 70820 Kuopio (FI); MYLLYMAA, Sami, 70820 Kuopio (FI); TÖYRÄS, Juha, 70100 Kuopio (FI)
(74) Representative: LEITZINGER OY
(86) International application number: PCT/FI2013/051054
(87) International publication number: WO 2014/072582

(56) References cited:
- WO-A1-03/057030
- WO-A2-01/30232
- JP-A- H10 201 725
- US-A1- 2008 146 958
- US-A1- 2009 105 577
- US-A1- 2009 247 894
- US-A1- 2010 036 275
- US-A1- 2012 083 673

## Description

### Field of the invention

The invention relates to the field of medical technology, more specifically to electroencephalogram (EEG) measurement.

### Prior art

By means of electroencephalography (EEG) is examined the electrical activity of the brain. Changes in the membrane stresses of neurons of the brain are recorded by means of electrodes attached to the patient's scalp. When changes take place simultaneously in several neurons, the voltage fluctuations in single neurons are summed and a measurable EEG signal is produced. In practice, a signal recorded from the scalp originates from simultaneous changes in the post-synaptic potentials of the pyramidal neurons of the cortex. The voltage fluctuation in an EEG is typically 5-250 µV and the frequency range 1-70 Hz.

EEG examinations are used especially in specialised neurophysiological units in diagnosing epilepsy. There, the EEG recording is carried out by specialist staff with high-level training and experience in using measuring devices and in the correct placement of the electrodes. However, EEG recordings would undoubtedly be useful also outside units specialising in EEG, for example, in intensive care, paramedic care and health centres, where by means of recordings could be detected various disorders in the electrical activity of the brain relating to, for example, severe brain damage, cerebral infarction, cerebral haemorrhage, subarachnoid haemorrhage, intoxication and unclear consciousness disorders. For example, in paramedic care, the electrical activity of the heart (ECG) is recorded routinely, but the electrical activity of the brain, the EEG, is recorded rarely. The reason for this are presumably the clumsy EEG sensor applications on the market, which are slow and difficult to place correctly on the patient without specialised training and extensive experience. The lack of EEG monitoring currently presents a central diagnostic challenge; for example in emergencies and in paramedic care, often hardly anything is known about the patient's brain activity or possible damage to the brain until the patient has been moved to a hospital and to EEG monitoring. It would be essential to be able to diagnose a dysfunction in the electrical activity of the brain as soon as possible and start the appropriate treatment as quickly as possible.

Currently, the EEG is most commonly measured by using an internationally standardised, so-called 10-20 system. As electrodes are most often used cup electrodes made of, for example, silver-silver chloride (Ag-AgCl), silver, tin, gold or platinum. The said 10-20 system has several disadvantages. The system uses 21 different electrodes, the positions of which in relation to the skull must be determined accurately, in order to be able to make a reliable diagnosis. A measuring wire connected to each electrode makes the measuring connection rigid and uncomfortable, hindering the patient's normal movements, and may cause interferences (movement artefacts) in the measuring signal and thus complicate the interpretation of the EEGs. Placing cup-like electrodes on the scalp requires preparation of the skin, that is, mechanical scraping of the skin to remove the dead surface layer (epidermis), and dosing of a conductive medium (electrode gel). Finally, the adhesion of the electrodes is ensured by different attachment systems, such as tapes, bands, nets, caps or adhesive fixing paste. The preparations for measurement thus require a lot of experience and special know-how. In addition, the said known equipment requires a considerable amount of time (30-50 min), thus delaying the initiation of the patient's proper treatment and considerably increasing the treatment costs.

Many of the current clinically used electrodes are not compatible with magnetic imaging (MRI) and computed tomography (CT) equipment, and thus the electrodes have to be removed from the patient to ensure safety and imaging quality. Removing and reattaching the electrodes cause a time-wise long interruption in the EEG recording, in which case a clinically significant abnormality in the EEG may remain completely undetected, leading in the worst case to an incorrect diagnosis. The removal and reattachment of the electrodes also cause the patient skin irritation, pain and a potential risk of skin infection.

In prior art document US2008091089A A1 is presented a single-use, self-contained device to monitor at least one physiological parameter of a subject. The device includes a physiological sensor to sense a subject physiological parameter and to generate a physiological signal. An integrated circuit is coupled to the at least one physiological sensor and processes the physiological signal. An indicator is electrically coupled to the integrated circuit and indicates information associated with the physiological parameter or the subject. A power source is electrically coupled to the physiological sensor and to the indicator. A housing carries the physiological sensor, the integrated circuit, the indicator and the power source. The device includes means for limiting the device to a single use.

Prior art document WO 2001/30232 A2 presents reusable appliance for acquisition from a patient of EEG signals comprising a micro-miniature, micro-power, low noise multi-channel data-acquisition system powered by a fiber optic illuminator and photovoltaic cell thus eliminating the need for inductively coupled power converters. The appliance also uses disposable EEG electrodes which are similar in size.

In prior art document US2009/105577 A1 is presented embodiments relating to a wireless physiological monitoring system. The system includes at least one wireless sensor and a monitoring device which are linked to one another wirelessly for measuring physiological signals of a patient. The at least one wireless sensor is located on the patient and may comprise a wireless surface electrode assembly or a wireless needle assembly.

In prior art document US2012083673 A1 is presented a sensor for monitoring the depth of consciousness of a patient. The sensor includes a plurality of light sources, light detectors, and in some embodiments, electrodes.

Prior art document WO 2003/057030 A1 presents a physiological sensor combination having a flexible substrate configured to attach to a tissue site. Multiple sensors are disposed on the substrate, which generate physiological signals. Each of the signals is responsive to a different physiological parameter. Conductors are carried on the substrate and routed between the sensors and at least one connector. The connector is configured to communicate the physiological signals to at least one monitor, which derives measurements of the parameters.

Prior art document US2009247894 A presents a system for acquiring and processing a subject's brain electrical activity. The system includes at least one electrode, at least one analog amplifier channel, an analog-to-digital converter, a stimulus generator, and a digital signal processor configured to perform a harmonic signal analysis algorithm. The at least one analog amplifier channel, the analog-to-digital converter, and the digital signal processor are configured on a single integrated physical circuit.

In the prior art document US2010036275 A1 is presented an improved apparatus and method of using an EEG net to obtain electroencephalographic measurements from a patient in an emergent or urgent care setting.

In scientific publication: Salmi T K et al: " Innovation in emergency neurology: recording of EEG with an EEG-to-ECG adapter " is presented an adapter to transfer EEG measurement signals to an EKG amplifier. The electrodes used in said publication are invasive three-dimensional spiral electrodes to be located under skin of a patient.

Commercially (e.g. in patent publication EP0951233B1) are available simple disposable electrodes which are adhered to the forehead, which typically have only a few (1-4) measuring channels and are mainly used for determining the depth of anaesthesia during operations. Due to the small number of electrodes, such solutions are, however, unsuitable for diagnosing disorders in brain activity (for example, epilepsy, coma, cerebral haemorrhages).

One prior art solution is a quick-to-use matrix electrode (StatNet^{™}, HydroDot Inc., WO2009/061920A1), which consists of two strips placed crosswise over the head. The strips have a sandwich structure with silver-silver chloride electrodes and silver signal transmission lines integrated in flexible plastic film. In each strip, the transmission lines end on the edge of the strip, from where the signals measured are led by means of a quick coupling to an amplifier. The strips are coated with an adhesive by means of which the sensor remains adhered to the skin by itself. On top of the electrodes is a porous pre-moistened pad construction. No preparation of the skin or other pre-treatment are thus required. The set-up time of the sensor is said to be 5 minutes and the operating time of the electrodes 4 hours. StatNet's sensor, for example, is placed over the hair and is structurally relatively rigid (bends only in the direction of the strip), conforms poorly to surfaces curving in several directions, moves easily with the hair, and requires monitoring to ensure that it has remained in place. This sensor application is also unsuitable for patients whose head or neck must not be moved or who have injuries or measuring instruments in the cranial area. The prior art implementation in question is suitable for paramedic and intensive care unit use, but not for several other EEG examinations, such as long-term epilepsy studies or sleep studies.

Patent publication US6032065A discloses an easy-to-use and disposable EEG matrix electrode for use in the hairless areas of the face. The substrate is preferably made of a non-conductive polymer, such as mylar. In the said publication, the ground electrode is located centrally, the reference electrode on the neck, and the movement of the eyes and the EMG signal from the chin are monitored by means of the said electrodes. There are only two EEG electrodes on the temples. In this prior art implementation, a separate medium (electro-gel) has to be used to be able to affix the electrodes on the surface of the skin and to form a proper electrode contact.

Patent publication US2010/0041962A1 discloses a matrix electrode intended for EEG monitoring, which comprises electrode contacts placed on the hairless areas of the face. It shows a sensor construction which makes possible lateral extension of the sensor in order for the sensor construction to fit the different face sizes of patients. The mutual placement of the electrodes with respect to one another changes when attached to faces of different size. In addition, stable attachment of the sensor construction is ensured with a separate adhesive layer. The attachment of the sensor construction is ensured with a structure extending behind the ear.

Patent publication JPH10201725A relates to reducing eddy current generated in an electrode for an organism put on a fluctuating magnetic field and to reduce a heat generated by the eddy current.

Each of the above mentioned prior art documents fails to present an electrode configuration of spiral structure being in contact with hairless skin area through an electroconductive active attachment layer for carrying out electrode measurements, and a grounding layer formed completely or partly over the body part to prevent the effect of external electrical interferences by isolating the grounding layer with an isolation layer from the active attachment surface and the means for transmitting the measurement data to provide an essentially good signal-to-noise ratio (S/N).

### Brief description of the invention

The invention is defined by the appended claims.

The aim of the invention is to eliminate and reduce the problems relating to the prior art EEG electrode solutions disclosed, which relate to the difficult and slow placement of the electrodes, to instable skin contact, to the drying of the electrodes (in other words, increase in measuring resistance, that is, impedance), to noise and interference levels, and to incompatibility with magnetic imaging (MRI) and computed tomography (CT) equipment. In other words, the aim of the invention is to realise an electrode measurement implementation for recording the electrical activity of the brain on the surface of the skin of the patient's head, by means of which implementation electrode measurements are substantially facilitated and speeded up to produce high-quality measurement data for examination or monitoring.

The object of the invention is achieved by a matrix electrode configuration for carrying out electrode measurements on the surface of the skin of a patient's head for recording the electrical activity of the brain. The matrix electrode configuration comprises a body part of non-conductive material conforming to the contours of the surface of the skin, electrodes connected to said body part for producing measurement data, transmission lines for transmitting the measurement data to be received to a measurement data unit during use in order to further process the measurement data, and an active attachment surface configured to be located between the electrodes and the surface of the skin. The body part comprises an electrode placement configuration for maintaining the mutual placements of the electrodes essentially the same with respect to one another, and the active attachment surface comprises hydrogel or other electroconductive adhesive material which adheres well to the skin in order to form a stable and essentially interference-free attachment contact between the electrodes and the skin surface.

The electrodes and the transmission lines are made of conducting wires forming a conducting layer on a surface of to the body part. The conducting wires forming the electrodes define spiral shapes for the electrodes. The electrodes are suitable to be in contact with hairless skin area through the electroconductive active attachment layer. The matrix electrode configuration also comprises an insulator coating layer on the body part covering the conductor layer to avoid short-circuiting of the wires. The insulator coating layer is provided with openings at the electrodes. The matrix electrode configuration also comprises a grounding layer formed completely or partly over the body part to prevent the effect of external electrical interferences. The grounding layer is isolated from the active attachment surface and the conducting layer by the insulator coating layer.

An advantage of the invention is that rapid and reliable attachment of the matrix electrode configuration is provided in the hairless areas of the patient's head in such a way that the real mutual placement of the electrodes is known. Both the successful electroconductive attachment of the electrodes and the correct and desired placement of the electrodes make it possible to produce high-quality measuring information.

The matrix electrode configuration according to the present disclosure may be used in an arrangement for carrying out electrode measurements on the surface of the skin of a patient's head for recording the electrical activity of the brain. In addition to the matrix electrode configuration, the arrangement may comprise a measurement data unit for receiving the measurement data transmitted by the transmission lines of the matrix electrode configuration in order to further process the measurement data.

In this context, the word "hair" is supposed to mean only the hair growing from persons head, which can grow into length of dozens of centimetres if not specifically cut. This term should not be understood to mean the fluffy skin hair around the human body, neither is it supposed to mean any facial hair or other male or female typical hair growth on the skin. It is to be understood, that the hairless areas of the skin may in this context include skin hair or facial hair.

### List of figures

- Figure 1: shows the preferred placement of the electrodes of the matrix electrode configuration in the hairless areas of the patient's head.
- Figure 2: shows a preferred embodiment of the invention of an arrangement for carrying out electrode measurements.
- Figure 3: shows an example of a spiral electrode configuration made with printing technique.

### Detailed description of the invention

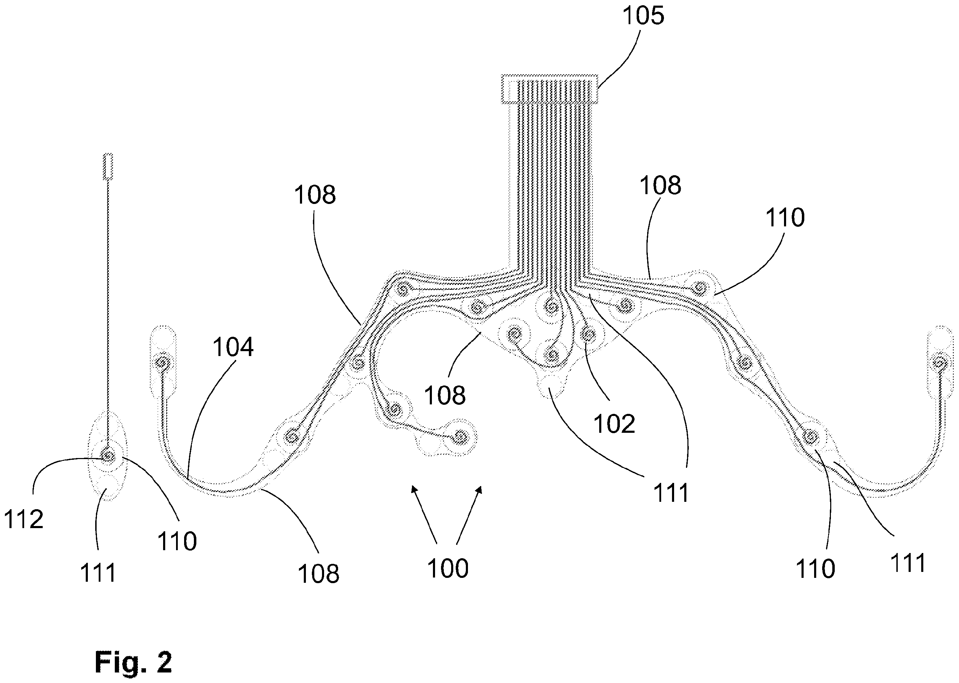

In the arrangement according to the present invention for carrying out electrode measurements, electrical activity of the brain is measured on the surface of the skin of the patient's head to produce measurement data. The measurement data is stored for assessment of the electrical activity of the brain. Figure 2 shows one preferred embodiment according to the invention of an arrangement for carrying out electrode measurements. The arrangement comprises a matrix electrode configuration, which comprises a body part 100 of non-conductive material conforming to the contours of the surface of the skin. To the said body part 100 are connected electrodes 102 for producing the said measuring data. The means 104 for transmitting the measurement data are also connected to the said body part 100. The means 104 are, for example, conductor lines, for example, to a ZIF-type quick coupling 105 through which the measurement data is transmitted further through a wire or wirelessly to a measurement data unit for further processing of the measurement data. The measurement data unit is, for example, a computer unit for diagnostic examinations.

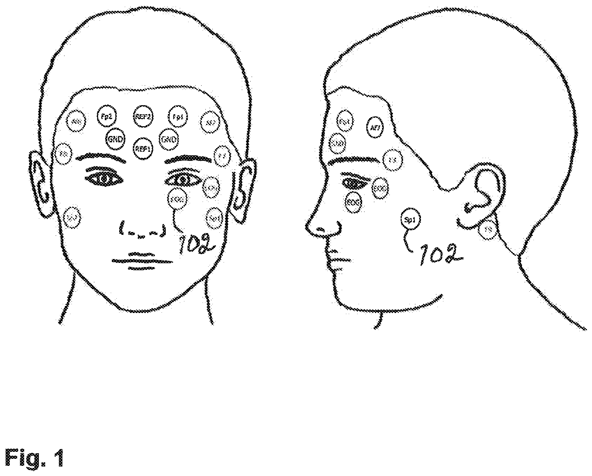

Figure 1 shows the preferred placement of the electrodes of the matrix electrode configuration in the hairless areas of the patient's head. The body part 100 (Figure 2) according to the invention comprises an electrode placement configuration 108, by means of which the mutual placements of the electrodes 102 are maintained essentially the same with respect to one another. The arrangement according to the invention also comprises an active attachment surface 110 located between the electrodes 102 and the surface of the skin for forming a firm and electroconductive attachment between the electrodes and the surface of the skin, by means of which attachment contact are transmitted measurable signals from the patient's head to the electrodes through the said electroconductive active attachment surface 110 for producing measurement data. The active attachment surface may also comprise an area or areas 111 which are of non-conductive material which adheres to the skin, which comprises hydrogel or other substance or component with the said similar properties.

In a preferred embodiment of the invention, the body part 100 comprises the said electrode placement configuration 108 for easy attachment of the matrix sensor construction in the hairless skin areas of the patient's head by means of the said active attachment surface 110. In this case, each electrode attaches to its intended measuring point in the said hairless skin areas of the head. The electroconductive active attachment surface 110 preferably comprises hydrogel in order to form a stable and essentially interference-free attachment contact between the electrodes 102 and the skin surface. Completely or partly over the body part 100 is formed a grounding layer to prevent the effect of external electrical interferences. The grounding layer is isolated by an isolation layer and/or the body part from the active attachment surface 110 and the wires. If there are no holes in the body part, it may act as isolation. If there are holes in the body part, isolation can be provided by a separate layer. The grounding layer functions by preventing the induction of electrical interferences to the wires and thus to the measured EEG signal, that is, the measurement data.

The matrix electrode configuration may preferably comprise at least two reference electrodes 102 and preferably at least two ground electrodes 102, between which can, when measuring, be selected which electrode combination is used to produce the measurement data. If the electrode contact is good, the matrix electrode configuration may also comprise only one reference electrode and only one ground electrode. The grounding layer is preferably connected to the ground electrode or ground electrodes. Matrix electrode configurations may be scaled to fit optimally different sizes of heads without the scaling affecting the number of electrodes used. The arrangement according to the invention may comprise at least the matrix electrode configuration packed into a package, on the outside of which can be seen dimensioning indicators for ensuring the correct matrix electrode configuration size to fit the patient's head without opening the package.

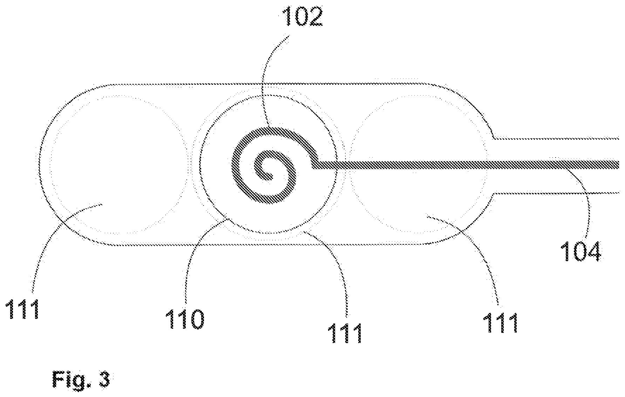

The signal-to-noise ratio (S/N) can be improved by forming and using an optimised electrode configuration to provide an essentially good signal-to-noise ratio (S/N), as well as MRI and CT imaging compatibility. An optimised electrode configuration is, in the context of the claimed invention, a spiral matrix electrode configuration. Figure 3 shows an example of a spiral electrode configuration 102 produced by printing technique. The area 111 surrounding the electrode 102, and the separate circular areas 111 in the examples according to Figures 2 and 3, represent areas 111 of the active attachment surface with non-conductive material attaching to the skin surface, the material comprising hydrogel or other substance or composition with the said corresponding properties. In addition, the arrangement for carrying out electrode measurements may comprise at least one electrode 112 attached in the patient's chest area for carrying out cardiac measurements (ECG).

In the following are described, with reference to Figures 1-3 or at least some of them, different possible uses and embodiments of the invention. From here onwards, the matters disclosed in this detailed description are thus examples of the various possible implementations and properties of the different parts 100, 102, 104, 105, 108, 110, 112 of the invention. According to the present invention is thus realised a matrix electrode configuration comprising a body part 100 conforming to the contours of the face, which body part is made of non-conductive material, for example polyester film, and to which the electrodes 102 and the measurement data transmission wires 104 are integrated. Thanks to the body part, the positions of the electrodes with respect to one another remain essentially unchanged and positioning the electrodes in the correct places on the patient's face and also elsewhere is easier. The electrodes fixed to the body part are placed in the hairless areas on the patient's forehead, temples, cheeks and bridge of the nose, as well as behind the ears. One electrode may in addition be placed on the chest for ECG monitoring.

The matrix electrode is designed to be attached directly onto cleansed skin and to cover all essential EEG measurements normally made in hairless head areas. Separate gels or electrode pastes are not needed and may not be used for attachment. The active attachment surface based on hydrogel immediately provides a stable contact with the skin which may last for days. From the point of view of manufacturing technique and structure, the matrix electrode is relatively simple and economical and thus suitable to be disposable. The matrix electrode is delivered sterilised in a disposable package.

In one exemplary matrix electrode configuration, as wires 104 is used silver-plated Aracon fibre. The electrodes 102 are spiral (diameter 7 mm) structures made of silver wire, which are coated with an electroconductive hydrogel film. The matrix electrode comprises a total of 16 electrodes, of which 10 are EEG measuring electrodes (in the Figure Fp1, Fp2, Af7, Af8, F7, F8, Sp1, Sp2, T9 and T10), 2 are EOG electrodes (identification of eye movements, in the Figure "EOG" and "EOG"), 2 are reference electrodes (REF), and 2 are ground electrodes (GND). The electrodes 102 may be spiral structures (102, Figure 3) at the end of a conductive metal wire or fibre (e.g. silver-plated isolated Aracon). As wire material can be used highly electroconductive material, such as silver, silver silver-chloride, gold or various alloys. A good contact of the electrodes with the skin is ensured with an electroconductive, for example hydrogel film, developed for medical use (e.g. AG602, Amgel Technologies). Skin attachment may, if necessary, be secured at desired points with skin tape. As support for the electrodes is used a thin film conforming to the contours of the face, which film may be, for example, polyamide, polyimide (Kapton^{™}) polyester (Mylar^{™}), or other elastic material.

The electrodes according to the invention are designed to be attached directly onto cleansed skin and to cover all essential EEG measurements normally made in hairless head areas. Separate gels or electrode pastes are not needed and may not be used for attachment. The active attachment surface based on hydrogel immediately provides a stable contact with the skin which may last for days.

The matrix electrode configuration according to the invention may be formed, for example, by a method of implementation based on lamination technique. The sensor implementation has a sandwich structure, comprising, for example, the following layers when viewed from the outside towards the patient's skin: stiffener layer (1), grounding layer (2), base layer (3), conducting layer (4), insulator layer (5), hydrogel layer (6), release liner layer (7). As the body part (base layer) of the matrix electrode is used a thin polymer film, such as Mylar^{™}, which conforms to the contours of the face. The electrodes 102 and their transmission lines 104 (conducting layer) are thick-film structures made of conductive ink (e.g. Ag/AgCl ink) on the surface of the mylar. The body part of the matrix electrode is coated with insulating material (insulator layer) to avoid short-circuiting of the wires. In the insulating material are openings at the electrodes, through which the electrode spirals are in contact with the hydrogel (for example, AG602, Amgel Technologies, Fallbrock, CA, USA). The release liner layer is a protective film layer which protects the electrodes from drying; the film may be, for example, a thin plastic film, which is torn off at the stage when the attachment of the electrode is begun.

All transmission lines preferably end in the sensor's projecting part, from which the measured signals are guided by means of a quick coupling to an amplifier. As a coupling is used either an MRI-compatible coupling or an easily disconnected/reconnected coupling (e.g. so-called ZIF-type connector). The conductor layer is covered by an insulating layer (insulator layer) with oval openings at the spiral electrodes. A good contact of the electrodes with the skin is ensured by areas of hydrogel film attached at the electrodes. As a booster is in addition used non-conductive hydrogel around each electrode (hydrogel layer or other adhesive material) to facilitate the placing of the extremely flexible sensor on the patient's skin and to ensure good attachment. The hydrogel layer is covered with a protective layer (release liner layer) which gives support during the mounting of the electrode. The matrix electrode comprises a total of 17 electrodes, 10 of which are EEG measuring electrodes, 2 are EOG electrodes (identification of eye movements), 2 are ground electrodes (GND), 2 are reference electrodes (REF) and 1 is an ECG electrode (measurement of the electrical activity of the heart) placed on the chest.

In view of the exemplary implementations 1 and 2, it is obvious to a person skilled in the art that the matrix electrode described in the invention can be implemented with very different techniques, for example, with thin film and lithographic methods, silk-screen printing technique, printing techniques, various lamination techniques, etc.

The arrangement according to the invention for carrying out electrode measurements can be utilised and used, for example, in the following applications and with them can be achieved, for example, the functional improvements disclosed in the examples:

### Example 1. Short-term monitoring, advantage of rapid reliable attachment of headset, no special skills required

An unconscious patient is brought to an emergency department. A paramedic is able to attach the invention on the patient's forehead and face within a few minutes, without separate EEG nurse training. Thus, a general idea of the state of the patient's brain is obtained quickly. Changes in the EEG due to possible conditions requiring emergency care (e.g. status epilepticus, subarachnoid haemorrhage, infections in the central nervous system, as well as metabolic and toxicological disorders) are detected immediately and the patient can be referred to the appropriate treatment as quickly as possible.

### Example 2. Long-term monitoring

The invention is used in a hospital ward for long-term EEG monitoring to be able to detect random abnormalities in a patient's EEG in the long term. In long-term monitoring can at the same time be detected possible sudden deterioration of the patient's neurological status. The electrodes according to the invention withstand long-term recording without drying, and no allergic reactions appear on the skin. The matrix electrode is lightweight, flexible and breathable and does not, therefore, cause the patient additional stress. Measurements were carried out with a Telefactor electroencephalograph and the contact impedances of the electrodes and the signals measured maintained a high quality throughout the 48 hour measurements.

### Example 3. Status epilepticus

Status epilepticus (SE) is a life-threatening neurological state of emergency which requires fast and efficient treatment. SE is caused by excessive excretion of excitatory transmitters (glutamate) from their nerve endings. It is estimated that as many as 20% of the discharges are non-convulsive, that is, do not cause observable convulsions. Therefore, without EEG measurement, a patient may be suffering from epileptic discharge activity without it being observed and treated. The invention provides a fast and easy way of verifying whether an unconscious patient is in a non-convulsive state. The matrix electrode can also be used during the loading of status epilepticus medication, in which case any cerebral or cardiac complications can be detected immediately. In addition, the matrix electrode can be used for monitoring the burst decay state used in the treatment of status epilepticus. The burst decay can be seen extremely well from the forehead electrodes.

### Example 4. SAH patient (subarachnoid haemorrhage)

An SAH patient is bleeding into the space between the arachnoid and the pia mater immediately surrounding the brain. A sudden severe headache is the single most significant symptom of subarachnoid haemorrhage. Partly due to the lack of suitable, easy-to-use EEG electrodes, EEG monitors are not generally used in monitoring the status of SAH patients, although this has been reported as being of primary importance for successful treatment. The invention provides a fast and easy way of carrying out the monitoring of an SAH patient.

### Example 5. Damage to cranium or cervical spine, normal EEG not suitable; one example of intensive care

The patient was found unconscious in the street and has, or is suspected to have, sustained an injury to the cranium or cervical spine. Normal EEG is not suitable because a conventional measuring connection cannot be attached on the injury. Also, due to the injury, the patient's head may not be moved and placing a conventional measuring connection would require that. The invention makes possible EEG measurement of this type of neck/head injury patient without having to move the patient's head or place the electrodes on the injury.

### Example 6. Craniotomy

Craniotomy refers to a surgical procedure in which the skull is opened to gain access to the brain. Craniotomy is also used for treating cerebral edema and increased intracranial pressure to reduce the pressure and give the brain room to swell. Different types of epileptic phenomena are common in the EEG in this type of treatments. Using a normal EEG connection is, however, impossible, because electrodes cannot be placed on the craniotomy. An easy-to-use matrix electrode fixed on the forehead and face makes EEG monitoring of the of this type of patient possible without endangering the patient's health.

### Example 7. Isolation patient (disposability, safety and ease of use)

A patient is suspected as having the Creutzfeldt-Jacob disease (CJD, commonly known as the "mad cow disease") and is in isolation. CJD causes distinct changes in the EEG signal, and thus measurement is extremely useful in identifying the disease. The invention speeds up and facilitates the measurement of such isolation patients. The invention also reduces the risk of contamination because it is disposable and quick and easy to attach. The matrix electrode can be destroyed immediately after the recording.

### Example 8. Patient has measuring instruments connected through the skull into the brain, normal EEG is unsuitable

In the intensive care unit, a patient may have various measuring devices (for example a microdialysis device or intracranial pressure meter) connected through the skull directly into the brain. In this case, it is impossible to carry out normal EEG measurement. By means of the invention measurement can, however, be carried out, because it is placed on the patient's forehead and face instead of on the skull. Measurement carried out with the invention does not interfere with the other measuring devices and no risks are caused to the patient, because the measuring devices connected to the central nervous system do not have to be moved.

### Example 9. Monitoring following resuscitation

When the heart stops or circulation to the brain is otherwise disrupted, there is always a risk of the patient sustaining temporary or permanent hypoxic ischemic encephalopathy due to lack of oxygen in the brain. Since the invention is easy and quick to attach, it provides a quick solution to EEG monitoring following resuscitation. By means of the invention, information on possible injuries is obtained already within a few minutes following the resuscitation. The invention could be a standard accessory included in the resuscitation equipment so that it could always be placed immediately after resuscitation.

### Example 10. PLED (periodic lateralized epileptiform discharges)

In connection with severe brain damage, in the EEG signal can be observed PLED waves, which appear periodically. In some cases, a PLED wavelet may be confused with an ECG artefact and thus it is good to have also the ECG signal displayed next to the EEG. In contrast to other instant EEG solutions, our invention includes a separate electrode for the ECG, which thus measures the electrical activity of the heart.

### Example 11. Sensor for depth of anaesthesia

Since the invention is easy and quick to use and in addition disposable, it can also be used for recording the EEG signal for the purposes of an anaesthesia monitor. The invention is connected by means of a suitable adapter to an anaesthesia monitor and the depth of the anaesthesia can be read directly on the monitor's display using an electrode intended especially for the said use. Since the invention is versatile, it is not necessary to buy several different sensors suitable for EEG measurement for intensive care units. In addition to the matrix headset according to the invention are provided various adapter connectors, by means of which the headset can easily be connected to different types of EEG amplifiers and monitors.

### Example 12. MRI compatibility (phantom measurement)

The arrangement according to the invention is not ferromagnetic. The MRI compatibility of the invention has been tested on an MRI phantom. The arrangement according to the invention does not warm up in an MRI imaging device or cause interference in MRI images. Therefore, the invention does not have to be removed for the duration of magnetic imaging, and EEG measurement can be continued immediately after the imaging. Normally, the EEG sensors have to be removed for the duration of the imaging, after which they have to be reattached if recording is to be continued. This takes up extra time and may slow down the patient's referral to the appropriate care. In addition, the reattachment of the electrodes always involves a risk of abrading the skin, which in turn increases the risk of sustaining different allergic reactions or infections. The MRI compatibility of the invention is ensured with a disconnectable ZIF-type coupling, which is easy to remove before imaging and the connection with the amplifier is reinstated by reattaching the ZIF coupling to the matrix electrode.

### Example 13. CT compatibility, phantom

The compatibility of the invention with computed tomography imaging (CT) has been tested with a phantom. The invention does not warm up in the CT device or cause significant interference in the images taken. Therefore, the invention does not necessarily have to be removed for the duration of CT imaging, and EEG measurement can be continued immediately after the imaging. Normally, the EEG sensors would have to be removed for the duration of the imaging, after which they have to be reattached if recording is to be continued. This takes up extra time and may slow down the patient's referral to the appropriate care. In addition, the reattachment of the electrodes always involves a risk of abrading the skin, which in turn increases the risk of sustaining different allergic reactions or infections.

### Example 14. Ambulance or ambulance helicopter measurements

Since the invention is extremely easy to use, it can be used to carry out EEG recordings in field conditions, already in an ambulance or ambulance helicopter. In practice it is appropriate to attach the matrix electrode to the patient in the ambulance, ambulance helicopter or in the army at a dressing station or in a field hospital. In this way, the state of the patient's brain and possible dysfunctions are known already before the patient arrives at the hospital. The EEG data is also sent wirelessly to the hospital's data system. The patient can thus be referred to the appropriate care and be started on the appropriate medication immediately on arrival at the hospital.

### Example 15. Wireless data transfer solutions

The invention is extremely light, easy to place and disposable. It is, therefore, highly usable as an EEG sensor for wireless EEG measuring solutions intended for various field solutions. Wireless solutions can be implemented by Bluetooth, wlan, GSM, 3G or infrared techniques.

### Example 16. EEG use at health centre level, transmission of results to central hospital

Since it is easy and quick to carry out EEG measurement by means of the invention, and no special skills are required to place it, carrying it out is easy also for health centre nurses. The EEG can thus be measured immediately already at the health centre's on-call service without having to refer the patient to a central or university hospital for the recording. A neurophysiologist can read the EEG remotely through the Internet and the patient can be immediately referred to the appropriate care. This is important, because in many cases early treatment improves the prognosis for recovery. With recordings at health centres are potentially also saved considerable sums in transportation costs since the patients do not necessarily have to be transported from one hospital to another.

### Example 17. Monitoring of premature infants

With very small premature infants, so-called amplitude-integrated EEG (aEEG) monitoring is started immediately after birth in order to be able to detect possible epileptic activity. As electrodes are currently used needle electrodes, which are quick and easy to attach. However, when attaching them, the skin has to be pierced, which again increases the risk of sustaining different inflammations or infections. If the invention, which is equally quick and easy to attach, was used instead of needles, the risk of inflammations and infections could be significantly reduced. It is possible to make small versions of the invention to also fit premature infants.

### Example 18. Paediatric patients

Placing a conventional EEG connection on the head of an uncooperative child is a time-consuming, difficult and sometimes even an impossible task. In placing a conventional connection, the child's head usually also has to be scraped with a wooden applicator, which the child will find unpleasant. A child may, in addition, have a fear of needles and will be afraid of being pricked when the nurse takes the scraping applicator out. Sometimes even sedatives have to be used to be able to attach the conventional connection. This is obviously undesirable because sedatives also always affect the EEG signal to some extent. Placing the invention on the skin does not require scraping and thus cooperation with the child is much easier. Furthermore, placing it takes much less time and thus the child's patience lasts longer.

### Example 19. Chronic patients in health centres

In health centres, there are many chronically ill elderly patients with considerable difficulties in communication, whose neurological status has not been assessed. The invention would provide a quick and easy way of measuring these patients by routine screening and to assess their status. It is possible that some of them are, for example, in a non-convulsive epileptic state.

### Example 20. Alternative ground and reference electrodes

Since one has to work in difficult conditions in paramedical situations, where a patient may be, for example, very dirty or restless, there are two ground and reference electrodes in the invenion. If one of the two is broken, it can be programmatically replaced by an unbroken one and thus the functioning of the EEG recording can be ensured.

### Example 21. Solutions facilitating use contained in the package

The headset package is an essential part of the product facilitating its use. It may contain, for example, clear illustrated numbered instructions, a picture of the typical placement of the headset on the face, the means needed for cleansing the face (wet wipes, cleansing tape,...), the means for tying up the hair (if the hair would otherwise be in the way, e.g. net, headset, fastening clips,...), a coupling, etc. On the outer surface of the package can be provided, for example, a measuring scale, to function as a measuring indicator. It can be used (by trying the package on the patient's face), without opening the package, to estimate whether the said model is suitable or whether a different modular size should be selected. This helps in selecting the right size.

### Example 22. Impedance testing of different electrode heads on the skin

Preparation: in all cases cleansing with alcohol, light scraping with tape and moistening with 0.5% NaCl. As an example is presented a silver chloride cup electrode. The measurements of this exemplary implementation were carried out with a Telefactor EEG device. In this exemplary implementation, the spiral electrode gave the smallest contact impedance and the noise level of its signal was distinctly lower than with the other solutions.

The electrode implementation according to the invention, which attaches to the skin, can also be implemented by means of a suitable electroconductive adhesive. Such adhesives may be, for example, starch- and agar-based materials to which, for example, NaCl has been added to enhance electroconductivity.

### Example 23. Comparison of interference levels

The electrode used in the invention has been found to provide a better signal-to-noise ratio than prior art electrodes. This is due to the choice of materials used, the active attachment surface 110 used comprising, for example, hydrogel and different shapes/patterns of the silver silver-chloride electrode (disc, elliptic disc, ring, loop, finger-like shape, snake-like shape, spiral shape, etc.). The electrode is also extremely stable, and not sensitive to interference caused by the patient's movements. These are important properties in order to be able to detect the minute changes shown in the EEG.

In the following is described in detail a preferred embodiment of the invention, that is, a new type of disposable matrix electrode for monitoring electrical activity of the brain (EEG), which can be utilised especially in paramedic care situations. In an acute situation, measuring an unconscious patient's EEG is of essential importance in assessing the patient's status. In paramedic care, the electrical activity of the heart (ECG) is measured routinely, but the electrical activity of the brain rarely. The reason for this are the clumsy sensor implementations on the market, which are slow to place properly on the patient. With an EEG could, however, be detected various brain dysfunctions relating to, for example, severe brain damage, cerebral infarction, cerebral haemorrhage, subarachnoid haemorrhage, status epilepticus, intoxication and unclear consciousness disorders. It would, therefore, be essential to be able to diagnose brain-related disorders as early as possible and to initiate the relevant care quickly. The currently widely used EEG electrodes are metal electrodes (SS, Ag-AgCl, Pt), which are separate and adhered to the skin. The positions of the electrodes with respect to the skull must be determined accurately to be able to make a reliable diagnosis. This obviously requires special skills. Together, these factors complicate and slow down the starting of the EEG recording and it is, therefore, rarely carried out in the field.

The body part of one preferred embodiment of the invention is made of polyester film (Mylar^{™}). The electrical electrodes are silver spirals (diameter 7 mm) connected to the end of conductive silver-plated insulated Aracon fibre. A good contact between the electrode and the skin is ensured by a hydrogel film (diameter 12-18 mm) developed for medical use. To facilitate skin attachment, a breathable tape part is attached around each electrode. By means of this matrix electrode can be achieved the following advantages and improvements: a significantly lighter and more flexible solution with conforms better to the contours of the face and does not fold the skin; a stable electrode contact with the skin due to the highly adhesive hydrogel, and in this example also the skin tape support. This solution does not require strong compressive forces against the skin - as the first prototypes did - to function well; the electrical contact of the electrode is good and gel is not needed and may not be used; the contact between the silver wire and the gel is optimised by means of the form and spiral structure of the wire; better breathability is essential, especially with strongly perspiring patients; strongly attaching and extensive reference electrodes in the centreline; vertical and horizontal electrodes for eye movement for detecting artefacts; and electrodes attached behind the ears to improve monitoring at the back of the skull.

The invention provides many alternative solutions regarding manufacturing techniques, choice of materials, number of electrodes and location of electrodes. The above-mentioned, at present latest development version, is quite optimal concerning the choice of materials and their functionality. Since the size and measurements of people's skulls vary a great deal, an optimal solution takes also these into account and the sensor implementation can easily be made in a few basic sizes. Finding the correct position for the reference electrodes (here two electrodes) successfully facilitates the analysis of the measuring data considerably by eliminating various artefacts, which include, for example, confusing the electrical activity of muscles (EMG) due to eye movements with a measurable signal. According to our understanding, the type of electrode configuration according to the invention for the facial area (forehead, temples, bridge of nose) is not commercially available and thus the invention is new. The advantages of the invention include ease of use, inexpensiveness, disposability, simplicity and versatile reliable measurement.

In the following is further described a commercially viable solution representing a matrix electrode configuration according to the invention, where the matrix electrode configuration is a headset electrode configuration for implementing easy and rapid EEG monitoring. With this headset electrode configuration, or headset, are achieved solutions which facilitate the phased mounting of the headset. The protective films of the electrode attachment surfaces of the headset are divided into parts, and thus the headset can be attached in a controlled, phased manner on the face. The central forehead part is attached into place first, after which the other parts are attached on the right and left in phases, the furthest reaching parts last. By means of this headset electrode configuration are also achieved integrated solutions for good contact and prevention of drying of the headset electrodes. Outside the actual electrical electrode configuration, and in the spaces within the electrode configuration, are also parts adhering to the skin which secure the attachment. Around the electrodes is also non-conductive attachment material which facilitates attachment and for its part prevents the electrode from drying. The headsets include a quick coupling solution which makes it possible to connect a disposable sterile adapter to the headset. This coupling can be connected through a multipurpose adapter to different measuring devices. The headset is thus suitable for use, as a sterile solution with couplings, with various kinds of devices. The package of the headset is an essential part of the product which facilitates its use. It contains, for example, clear illustrated numbered instructions, a picture of the typical placement of the headset on the face, the means needed for cleansing the face (wet wipes, cleansing tape,...), the means for tying up the hair (if the hair would otherwise be in the way, e.g. net, headset, fastening clips,...), a coupling, etc. On the outer surface of the package is provided a measuring scale. By means of it can be estimated simply (by trying the package on the patient's face), without opening the package, whether the said model is suitable or whether a different modular size should be selected. This helps considerably in selecting the right size. On the EEG headset, that is, on the electrode configuration according to the invention, can be made a grounding layer isolated from the electrodes to diminish the induction of external electrical interferences to the wires and thus to the measured EEG signal.

Although the invention is described above in the specification with reference to the accompanying figures, the invention is not, however, limited to the specification or figures, but may be modified within the limits of the accompanying claims.

## Claims

1. A matrix electrode configuration for carrying out electrode measurements on the surface of the skin of a patient's head for recording the electrical activity of the brain, the matrix electrode configuration comprising
- a body part (100) of non-conductive material conforming to the contours of the surface of the skin,
- electrodes (102) connected to said body part (100) for producing measurement data, wherein the body part (100) has an electrode placement configuration (108) for maintaining mutual placements of the electrodes essentially the same with respect to one another,
- transmission lines (104) connected to said body part (100) for transmitting the measurement data to be received to a measurement data unit during use in order to further process the measurement data, and
- an active attachment surface (110) configured to be located between the electrodes (102) and the surface of the skin, said active attachment surface (110) comprising hydrogel or other electroconductive adhesive material which adheres well to the skin in order to form a stable and essentially interference-free attachment contact between the electrodes (102) and the skin surface,
**characterized in that**
- the electrodes (102) and the transmission lines (104) are made of conducting wires forming a conducting layer on a surface of a first side of the body part (100), wherein the conducting wires forming the electrodes (102) define spiral shapes for the electrodes (102), and wherein the electrodes are suitable to be in contact with hairless skin area through the electroconductive active attachment layer (110),
- the matrix electrode configuration comprises an insulator coating layer on the body part (100) covering the conducting layer to avoid short-circuiting of the wires, wherein the insulator coating layer is provided with openings at the electrodes (102), and
- the matrix electrode configuration comprises a grounding layer formed completely or partly over the body part (100) on a second side, opposite to the first side, of the body part (100) to prevent the effect of external electrical interferences, the grounding layer being isolated from the active attachment surface (110) and the conducting layer by the body part (100) and/or by an additional isolation layer.

2. A matrix electrode configuration as claimed in claim 1, **characterised in that** the active attachment surface comprises an electroconductive surface (110) and a non-conductive surface (111).

3. A matrix electrode configuration as claimed in claim 1, **characterised in that** the matrix electrode configuration has at least one electrode (112) configured to be attached in the patient's chest area for carrying out cardiac measurements (ECG).

4. A matrix electrode configuration as claimed in claim 1, **characterised in that** the matrix electrode configuration comprises at least two reference electrodes (102) and preferably at least two ground electrodes (102) between which a desired electrode combination for producing the measurement data can be selected.

5. A matrix electrode configuration as claimed in claim 1, **characterised in that** the matrix electrode configuration is compatible to be worn during MRI and CT imaging.

6. An arrangement for carrying out electrode measurements on the surface of the skin of a patient's head for recording the electrical activity of the brain, wherein the arrangement comprises
- a matrix electrode configuration as claimed in any of claims 1 to 5, and
- a measurement data unit for receiving the measurement data transmitted by the transmission lines (104) of the matrix electrode configuration in order to further process the measurement data.

7. An arrangement as claimed in claim 6, **characterised in that** the arrangement comprises a set of different sizes of matrix electrode configurations to fit the patients' different head sizes.

8. An arrangement as claimed in claim 6, **characterised in that** the arrangement comprises at least the matrix electrode configuration packed into a package, on the outside of which can be seen dimensioning indicators provided for ensuring the correct matrix electrode configuration size without opening the package.

## Patentansprüche

1. Matrixelektrodenkonfiguration zur Durchführung von Elektrodenmessungen auf der Oberfläche der Haut des Kopfes eines Patienten zur Aufzeichnung der elektrischen Aktivität des Gehirns, wobei die Matrixelektrodenkonfiguration umfasst:
- einen Körperteil (100) aus nichtleitendem Material, dessen Form an die Konturen der Oberfläche der Haut angepasst ist,
- Elektroden (102), die mit dem Körperteil (100) verbunden sind, zum Erzeugen von Messdaten, wobei der Körperteil (100) eine Elektrodenplatzierungskonfiguration (108) aufweist, um zu bewirken, dass die Platzierungen der Elektroden relativ zueinander im Wesentlichen die gleichen bleiben,
- Übertragungsleitungen (104), die mit dem Körperteil (100) verbunden sind, zum Übertragen der zu empfangenden Messdaten während der Verwendung an eine Messdateneinheit, um die Messdaten weiter zu verarbeiten, und
- eine aktive Befestigungsfläche (110), die dafür ausgelegt ist, zwischen den Elektroden (102) und der Oberfläche der Haut angeordnet zu werden, wobei die aktive Befestigungsfläche (110) ein Hydrogel oder ein anderes elektrisch leitendes Klebematerial umfasst, welches gut an der Haut haftet, um einen stabilen und im Wesentlichen störungsfreien Befestigungskontakt zwischen den Elektroden (102) und der Hautoberfläche zu bilden,
**dadurch gekennzeichnet, dass**
- die Elektroden (102) und die Übertragungsleitungen (104) aus leitenden Drähten hergestellt sind, die eine leitende Schicht auf einer Oberfläche einer ersten Seite des Körperteils (100) bilden, wobei die leitenden Drähte, welche die Elektroden (102) bilden, Spiralformen für die Elektroden (102) definieren, und wobei die Elektroden geeignet sind, sich durch die elektrisch leitende aktive Befestigungsschicht (110) hindurch mit einem unbehaarten Hautbereich in Kontakt zu befinden,
- die Matrixelektrodenkonfiguration eine Isolierbeschichtungsschicht auf dem Körperteil (100) umfasst, welche die leitende Schicht bedeckt, um ein Kurzschießen der Drähte zu vermeiden, wobei die Isolierbeschichtungsschicht mit Öffnungen an den Elektroden (102) versehen ist, und
- die Matrixelektrodenkonfiguration eine Erdungsschicht umfasst, die vollständig oder teilweise über dem Körperteil (100) auf einer der ersten Seite gegenüberliegenden zweiten Seite des Körperteils (100) ausgebildet ist, um die Einwirkung äußerer elektrischer Störungen zu verhindern, wobei die Erdungsschicht von der aktiven Befestigungsfläche (110) und der leitenden Schicht durch den Körperteil (100) und/oder durch eine zusätzliche Isolationsschicht isoliert ist.

2. Matrixelektrodenkonfiguration nach Anspruch 1, **dadurch gekennzeichnet, dass** die aktive Befestigungsfläche eine elektrisch leitende Fläche (110) und eine nichtleitende Fläche (111) umfasst.

3. Matrixelektrodenkonfiguration nach Anspruch 1, **dadurch gekennzeichnet, dass** die Matrixelektrodenkonfiguration mindestens eine Elektrode (112) aufweist, die dafür ausgelegt ist, im Brustbereich des Patienten befestigt zu werden, um kardiale Messungen (EKG) durchzuführen.

4. Matrixelektrodenkonfiguration nach Anspruch 1, **dadurch gekennzeichnet, dass** die Matrixelektrodenkonfiguration mindestens zwei Referenzelektroden (102) und vorzugsweise mindestens zwei Erdungselektroden (102) umfasst, aus denen eine gewünschte Elektrodenkombination zum Erzeugen der Messdaten ausgewählt werden kann.

5. Matrixelektrodenkonfiguration nach Anspruch 1, **dadurch gekennzeichnet, dass** die Matrixelektrodenkonfiguration damit kompatibel ist, während der MRT- und CT-Bildgebung getragen zu werden.

6. Anordnung zur Durchführung von Elektrodenmessungen auf der Oberfläche der Haut des Kopfes eines Patienten zur Aufzeichnung der elektrischen Aktivität des Gehirns, wobei die Anordnung umfasst:
- eine Matrixelektrodenkonfiguration nach einem der Ansprüche 1 bis 5, und
- eine Messdateneinheit zum Empfangen der Messdaten, die von den Übertragungsleitungen (104) der Matrixelektrodenkonfiguration übertragen werden, um die Messdaten weiter zu verarbeiten.

7. Anordnung nach Anspruch 6, **dadurch gekennzeichnet, dass** die Anordnung einen Satz von unterschiedlichen Größen von Matrixelektrodenkonfiguration umfasst, um sie an unterschiedliche Kopfgrößen der Patienten anzupassen.

8. Anordnung nach Anspruch 6, **dadurch gekennzeichnet, dass** die Anordnung mindestens die Matrixelektrodenkonfiguration umfasst, die in eine Verpackung verpackt ist, auf deren Außenseite Bemessungsangaben zu sehen sind, die dazu vorgesehen sind, die korrekte Größe der Matrixelektrodenkonfiguration sicherzustellen, ohne die Verpackung zu öffnen.

## Revendications

1. Configuration d'électrodes matricielle pour effectuer des mesures d'électrode sur la surface de la peau de la tête d'un patient afin d'enregistrer l'activité électrique du cerveau, la configuration d'électrodes matricielle comprenant
- une partie de corps (100) en matériau non conducteur épousant les contours de la surface de la peau,
- des électrodes (102) connectées à ladite partie de corps (100) pour produire des données de mesure, dans laquelle la partie de corps (100) a une configuration de placement d'électrodes (108) pour maintenir essentiellement les placements mutuels des électrodes les unes par rapport aux autres,
- des lignes de transmission (104) connectées à ladite partie de corps (100) pour transmettre les données de mesure à recevoir à une unité de données de mesure pendant l'utilisation pour le traitement ultérieur des données de mesure, et
- une surface de fixation active (110) configurée pour être située entre les électrodes (102) et la surface de la peau, ladite surface de fixation active (110) comprenant un hydrogel ou un autre matériau adhésif électroconducteur qui adhère bien à la peau afin de former un contact de fixation stable et essentiellement sans interférences entre les électrodes (102) et la surface de la peau,
**caractérisée en ce que**
- les électrodes (102) et les lignes de transmission (104) sont constituées de fils conducteurs formant une couche conductrice sur une surface d'un premier côté de la partie de corps (100), dans laquelle les fils conducteurs formant les électrodes (102) définissent des formes en spirale pour les électrodes (102), et dans laquelle les électrodes sont adaptées pour être en contact avec une zone de la peau sans poils à travers la couche de fixation active électroconductrice (110),
- la configuration d'électrodes matricielle comprend une couche de revêtement isolante sur la partie de corps (100) recouvrant la couche conductrice pour éviter un court-circuit des fils, dans laquelle la couche de revêtement isolante est pourvue d'ouvertures au niveau des électrodes (102), et
- la configuration d'électrodes matricielle comprend une couche de mise à la terre formée complètement ou partiellement sur la partie de corps (100) sur un deuxième côté de la partie de corps (100) opposé au premier côté pour empêcher l'effet d'interférences électriques externes, la couche de mise à la terre étant isolée de la surface de fixation active (110) et de la couche conductrice par la partie de corps (100) et/ou par une couche d'isolation supplémentaire.

2. Configuration d'électrodes matricielle selon la revendication 1, **caractérisée en ce que** la surface de fixation active comprend une surface électroconductrice (110) et une surface non conductrice (111).

3. Configuration d'électrodes matricielle selon la revendication 1, **caractérisée en ce que** la configuration d'électrodes matricielle comporte au moins une électrode (112) configurée pour être fixée dans la zone de la poitrine du patient pour effectuer des mesures cardiaques (ECG).

4. Configuration d'électrodes matricielle selon la revendication 1, **caractérisée en ce que** la configuration d'électrodes matricielle comprend au moins deux électrodes de référence (102) et de préférence au moins deux électrodes de terre (102) entre lesquelles une combinaison d'électrodes souhaitée pour produire les données de mesure peut être sélectionnée.

5. Configuration d'électrodes matricielle selon la revendication 1, **caractérisée en ce que** la configuration d'électrodes matricielle est compatible pour être portée pendant une imagerie IRM ou TDM.

6. Agencement pour effectuer des mesures d'électrode sur la surface de la peau de la tête d'un patient afin d'enregistrer l'activité électrique du cerveau, dans lequel l'agencement comprend
- une configuration d'électrodes matricielle selon l'une quelconque des revendications 1 à 5, et
- une unité de données de mesure pour recevoir les données de mesure transmises par les lignes de transmission (104) de la configuration d'électrodes matricielle pour le traitement ultérieur des données de mesure.

7. Agencement selon la revendication 6, **caractérisé en ce que** l'agencement comprend un ensemble de différentes tailles de configuration d'électrodes matricielle pour s'adapter aux différentes tailles de tête des patients.

8. Agencement selon la revendication 6, **caractérisé en ce que** l'agencement comprend au moins la configuration d'électrodes matricielle conditionnée dans un emballage, à l'extérieur duquel sont visibles des indicateurs de dimensionnement pourvus pour assurer la taille correcte de configuration d'électrodes matricielle sans ouvrir l'emballage.
